# EUROPEAN PATENT SPECIFICATION

(11) **EP 2 922 770 B1**
(45) Date of publication and mention of the grant of the patent: **02.11.2016**
(21) Application number: 13808300.1
(22) Date of filing: 20.11.2013
(51) Int. Cl.: B65D 83/54, A61M 15/00, B65D 83/30

(54) **METERED DOSE DISPENSING VALVE**
DOSIERVENTIL
VALVE DOSEUSE

(30) Priority: 23.11.2012 GB 201221063
(43) Date of publication of application: 30.09.2015
(73) Proprietor: 3M Innovative Properties Company, St. Paul, MN 55133-3427 (US)
(72) Inventor: STUART, Adam J., Bracknell Berkshire RG12 8HT (GB)
(74) Representative: Bergen, Katja
(86) International application number: PCT/US2013/070866
(87) International publication number: WO 2014/081746

(56) References cited:
- EP-A1- 0 567 348
- WO-A1-2013/188609
- FR-A1- 2 925 032
- GB-A- 1 206 518
- US-A- 3 620 421
- US-A- 3 675 832

## Description

### Cross Reference to Related Applications

This application claims the benefit of United Kingdom Application No 1221063.9, filed November 23, 2012, which is incorporated herein by reference in its entirety.

### Field

The present invention relates to metered dose dispensing valves and in particular to metered dose dispensing valves suitable for use with metered dose inhalers.

### Background

Asthma and other respiratory diseases have long been treated by the inhalation of appropriate medicament. Pulmonary inhalation is also becoming an attractive route of administration of medicaments that may be difficult to deliver orally such as proteins and peptides.

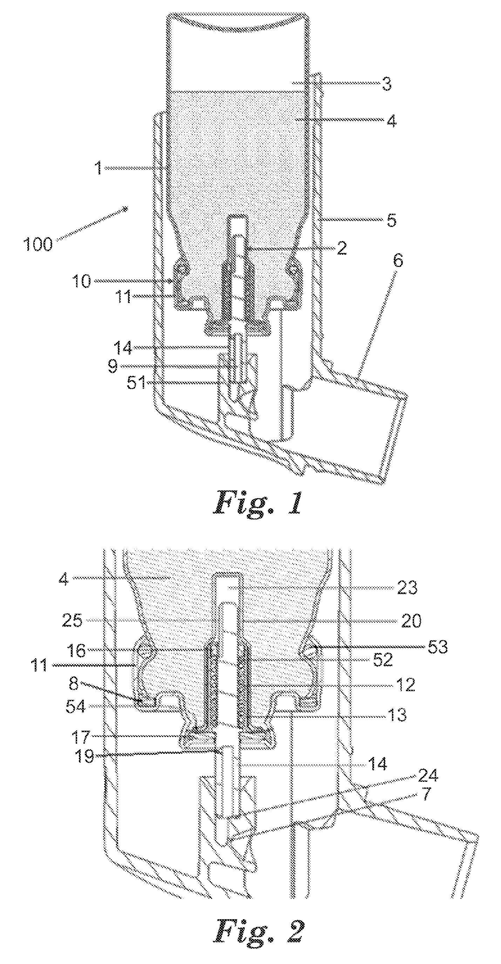

A widely used and convenient choice of pulmonary drug delivery has been the inhalation of medicament from an aerosol created by a pressurized metered dose inhaler (pMDI). As shown in Figure 1, a pMDI (100) typically comprises a canister (10) including a metered dose dispensing valve (2) mounted via a ferrule (11) onto an aerosol container or vial (1) defining in part a formulation chamber (3) filled with medicinal inhalation formulation (4), and an actuator (5) including a mouthpiece (6). (In an alternative form, suitable for nasal drug delivery, the actuator may comprise a nosepiece rather than a mouthpiece.) The canister is contained within the actuator by inserting the valve stem (14) of the valve, which protrudes outside the ferrule, into a support block (51) of the actuator. The valve stem has a dispensing passage (9) which allows for passage of substance from a metering chamber of the valve out through the valve stem and actuator nosepiece or mouthpiece to the user. Medicinal aerosol formulations typically comprise medicament either in solution or as particles suspended in liquefied propellant(s), e.g. CFC propellant(s) and more recently non-CFC propellant(s), such as 1,1,1,2-tetrafluoroethane (HFA134a) and/or 1,1,1,2,3,3,3-heptafluoropropane (HFA227). If desired and/or deemed necessary, the formulation may comprise other components, such as excipients, co-solvents, and suspending aids. Depending on the particular metered dose valve and/or filling system, medicament formulation may be filled into the pMDI either by cold-filling (in which chilled formulation is filled into the vial and subsequently the metered dose valve is fitted onto the vial) or by pressure filling (in which the metered dose valve is fitted onto the vial and then formulation is pressure filled through the valve into the vial).

Typical commercial pMDI metering valves comprise seven or eight or even more components. An example is the 3M Spraymiser™ valve by 3M Company, St Paul, Minnesota, USA (Figures 1 and 2). Referring to Figure 2, this valve in its usual form comprises eight (or optionally nine) components: a first valve body (13) defining in part a metering chamber (12), a second valve body (20) defining in part a pre-metering region (23) and acting in this valve as a bottle emptier, a valve stem (14), a biasing member in the form of a coil spring (52), an inner seal (16), an outer seal (17), a ferrule (11) and a gasket seal (8) (and an optional O-ring (53)). Although most of the individual components can be made cheaply, the number of them, together with a need for complicated manipulation during their assembly and a need for quite accurate alignment in their assembly, means that overall ex-factory costs can be relatively high, at least in terms of the prices that the rapidly developing markets of India, China and other eastern countries are willing to pay. This relatively high cost of valves may act as a barrier to their acceptance in highly price-sensitive markets. Accordingly marketing of associated pMDIs is limited, meaning many asthma sufferers in poorer countries are denied the common, accepted treatments that asthma sufferers would expect to receive in more affluent parts of the world.

Besides cost issues, it has been observed that issues related to component alignment and guidance and complex dimensional tolerance stack-up factors may generate secondary potential issues with valve and pMDI performance, such as poor pressure-filling performance, high firing forces and/or inadequacies in stem return forces, as well as undesirable propellant leakage and moisture penetration rates.

The following documents disclose types of valve, some of which might be intended to be low cost and/or to have a limited number of components: GB 1054307 (Riker), GB 2361228 (Groeger), US 3019947 (Gorman), EP 816 255 (Hildebrandt), GB 1115926 (Nadal), CH 428604 (Trautmann), GB 2300674 (Lacout), US 3521859 (Gronemeyer), US 3642180 (Lehmann), US 3862741 (Steiman & Beres), US 3982674 (Mildern), US 4471893 (Knickerbocker), US 4477001 (Galia), US 4852807 (Stoody), US 4887743 (Blake), US 5775545 (Sullivan) which discloses a valve according to the preamble of claim 1, WO95/28620 (Keller), and WO 02/02435 (Corba). Note that not all these valves are metering valves.

### Summary of Invention

Although a number of documents seemingly suggest low cost valves and/or valves with few components, presently there is no such valve with notable success on the market. Without wanting to be bound to a particular theory, it seems that the lack of success may be related to the fact that previously suggested valves show significant dissimilarities to industry-standard valves in the way that they interface with aerosol containers or actuator support blocks and/or perhaps in fact exhibit a higher level of complexity and/or lower levels of physical or operational robustness and/or provide poor valve performance (e.g. very variable dose volumes, high stem friction, high leakage rates, etc).

Accordingly there is an ongoing need and a demand for inexpensive pMDI valves of good quality and high reliability. It would be advantageous to overcome or mitigate issues in metering valves that can be made for very low cost, that can be pressure-filled, and that can interface in a conventional way to both the pMDI actuator and the typical aerosol container used in a pMDI (e.g. using a hollow male valve stem and a mechanically crimpable ferrule). In addition, it would be desirable to provide such a valve that would operate in the same way that conventional pMDI valves do: i.e. dispensing a metered dose of formulation along the valve stem bore when the stem is pushed inwardly (i.e. towards the aerosol container) along its longitudinal axis, and then resetting (with the stem moving back outwardly to its starting point) when the stem is released by the patient.

In one aspect of the present invention there is provided a metered dose dispensing valve for dispensing metered volumes of an aerosol formulation from an aerosol container, said valve comprising
a first valve body defining in part a metering chamber;
a compliant biasing member;
a valve stem passing axially through the metering chamber, movable relative to the chamber between non-dispensing and dispensing positions, and biased from its dispensing position towards its non-dispensing position by the compliant biasing member; and
a second valve body defining at least in part a pre-metering region;
wherein said valve stem and second valve body are integrally provided in a single component, wherein the biasing member is provided as a separate component and positioned relative to said valve-stem-second-valve-body-comprising component such that at least one portion of the biasing member engages the valve-stem-second-valve-body-comprising component at a first position, and at least one other portion of the biasing member engages the valve-stem-second-valve-body-comprising component at a second position, thereby imparting a bias to the valve stem, and wherein, in use, the valve stem is moved axially against said bias from its non-dispensing position into its dispensing position and upon release the valve stem moves under the action of the bias from its dispensing position back to its non-dispensing position.

Surprisingly we have found that by combining the valve stem and the second valve body into an integral single component and by positioning the biasing member (that is provided separately) relative to the integral component such that it imparts a bias to the valve stem, it is possible to provide a desirable metered dose dispensing valve for use in inhalation devices or for use in the provision of canisters (i.e. an aerosol container, filled or not yet filled with aerosol formulation, plus a valve) targeted for use in inhalation devices.

It will be appreciated that the at least one portion of the biasing member may engage the valve-stem-second-valve-body-comprising component at a location or alternatively, if e.g. two or more portions of the biasing member engage the valve-stem-second-valve-body-comprising component, at two or more locations (said location or said set of locations being generally referred to as the aforesaid "first position"), while the at least one other portion of the biasing member engages valve-stem-second-valve-body comprising component elsewhere, e.g. at a second location or at two or more other locations (said other location or other set of locations being generally referred to as the aforesaid "second position"). Favorably the at least one portion of the biasing member engages the valve-stem-second-valve-body-comprising component such that the at least one portion of the biasing member is anchored relative to the valve-stem-second-valve-body-comprising component.

Valves described herein are particularly desirable for dispensing metered volumes of pressurized aerosol formulations, and thus particularly suitable for use in pressurized metered dose inhalers or for use in the provision of canisters targeted for use in pressurized metered dose inhalers. In particular, the provision of such an integral single component comprising the valve stem and second valve body allows one to reduce manufacturing costs and/or avoid or minimize a number of performance issues and/or dimensional tolerance issues associated with valve assembly. Provision of a biasing member as a separate component allows force characteristics for the bias to be controlled precisely and simply, particularly where the biasing member is based on a standard coil spring, and to make adjustments to compensate for any changes made to the valve-stem-second-valve-body-comprising component e.g. when adapting a valve for use in a different product. In addition, valves described herein have a valve stem with axial travel distances to and from its dispensing and non-dispensing positions, wherein in its dispensing position a metered dose may be delivered and in its non-dispensing position the metering chamber may be refilled via the pre-metering region, said axial travel distances being very similar to those of industry-standard pMDI valves. Accordingly such valves are advantageously broadly similar to those of industry-standard valves, helping to make them readily compatible with existing actuators, aerosol containers and dose indicators, and making them acceptable to the industry as a stand-alone component offering.

Favorably, the second valve body comprises a non-compliant portion and a compliant portion. Desirably at least one part of the compliant portion of the second valve body is joined either directly or indirectly to the valve stem and at least one other part of the compliant portion of the second valve body is joined either directly or indirectly to the non-compliant portion of the second valve body. In this manner, an intermediate compliant portion is positioned between the non-compliant portion of the second valve body and the valve stem, which facilitates the movement of the valve stem relative to the non-compliant portion of the second valve body within the integral valve-stem-second-valve-body-comprising component. In addition the non-compliant portion of the second valve body can favorably act among other things as a fixed support and/or anchoring position for the compliant biasing member that in turn acts to apply a bias to the valve stem. Desirably the compliant biasing member is positioned with respect to the valve-stem-second-valve-body-comprising component, in particular the compliant portion of the second valve body, such that in use (e.g. actuating the valve) the compliant biasing member and the compliant portion of the second valve body can move in concert. In particular, favorably the at least one portion of the biasing member engages the valve-stem-second-valve-body-comprising component at a position proximate to or at the interface between the non-compliant and compliant portions of the second valve body towards the non-compliant portion and said at least one other portion of the biasing member engages the valve-stem-second-valve body component at a position proximate to or at the interface between the compliant portion of the second valve body and the valve stem towards the valve stem. For such embodiments it will be appreciated that since, as described above, the at least one portion of the biasing member is positioned relative to the valve-stem-second-valve-body-comprising component to allow for an anchoring of the biasing member and the at least one other portion of the biasing member is positioned relative to the valve-stem-second-valve-body-comprising component to impart a bias to the valve stem towards its non-dispensing position, generally said portions of the compliant biasing member do not engage the compliant portion of the second valve body per se, but engage the valve-stem-second-valve-body-comprising component near the interfaces between the compliant portion and non-compliant portion and between the compliant portion and valve stem, away from the compliant portion and towards the non-compliant portion and valve stem, respectively. More favorably, the biasing member is positioned such that the at least one portion of the biasing member engages the non-compliant portion of the second valve body at a position proximate to or adjacent to the interface between the non-compliant and compliant portions of the second valve body and said at least one other portion of the biasing member engages the valve stem at a position proximate to or adjacent to the interface between the compliant portion of the second valve body and the valve stem.

It is to be recognized that the compliant portion of the second valve body could be configured as to impart an additional bias onto the valve stem towards its non-dispensing position. Alternatively, the compliant portion of the second valve body could provide little or no bias onto the valve stem towards its non-dispensing position. For the sake of completeness, it is to be noted that the compliant portion of the second valve body could be configured as to impart a bias onto the valve stem towards its dispensing position. Generally this would mean that the compliant biasing member would need to be designed to provide a greater biasing force onto the valve stem towards its non-dispensing position than that which would otherwise be needed for the aforementioned alternative constructions wherein the compliant portion provides no bias or biases the valve stem towards its non-dispensing position.

To facilitate compactness in design, favorably one said portion of the second valve body is fully or partially contained within the other said portion of the second valve body, more favorably the compliant portion of the second valve body is fully or partially contained within the non-compliant portion of the second valve body. Desirably, the second valve body forms a cage-in-cage-like structure with the compliant portion being nested in the non-compliant portion.

Favorably, the valve operates similarly to industry-standard push-to-fire pMDI valves. Moreover, favorably the valve is configured and arranged such that, in use, when the valve stem is in its non-dispensing position, the pre-metering region is in communication either continuously or transiently with the contents of the aerosol container and the metering chamber is in communication at least transiently with the pre-metering region to allow substance to pass from the aerosol container to the metering chamber, and when the valve stem is in its dispensing position, the pre-metering region is isolated from the metering chamber and a communication path is provided between the metering chamber and the outside of the valve and aerosol container assembly, said path being either continuously or transiently open to allow substance to pass from the metering chamber to the outside of the valve and aerosol container assembly.

The skilled person will understand that the terms 'dispensing position' and 'non-dispensing position' actually each refer to a range of spatial positions of the valve stem over its span of axial movement. The term 'rest position' refers to one specific non-dispensing position. The terms 'continuously open' and 'transiently open' in the context of the communication path may refer to both the case where the communication path is open continuously or transiently spatially or is open continuously or transiently temporally during valve stem operation.

Favorably the valve is designed to be similar to industry-standard push-to-actuate pMDI valves in that, in use, the valve stem is moved axially, e.g. by the user or a breath actuated mechanism, inwardly towards the aerosol container from its non-dispensing position to its dispensing position and upon release moves outwardly under the action of the bias from its dispensing position to its non-dispensing position.

Integral single components including the second valve body and the valve stem may favorably comprise a polymeric material, for example either a single polymeric material or with one or more polymeric materials in a composite and/or including other materials such as fillers and/or reinforcing agents in one or more of the polymeric materials. Such components may be favorably molded, in particular via one-shot molding or two or more shot moldings.

The compliant biasing member may favorably be a spring member. The term "spring" is generally understood to be an elastic object (e.g. member) used to store mechanical energy, whereby when the object is compressed or stretched, the force it exerts is proportional (or approximately proportional) to its change in length. In particular, the compliant biasing member may favorably be a compression or a tension spring member. Alternatively, a torsion spring could be used.

Compliant biasing members may comprise metal or they may comprise a polymeric material. Alternatively they may comprise both metal and a polymeric material, for example when the compliant biasing member is made of metal with a polymer (e.g. low energy polymeric) coating or when the compliant biasing member includes a polymeric structure reinforced with a metallic sub-structure. The use of metal per se or a substructure of metal (or other suitable materials) may facilitate avoidance or reduction of any tendency of the compliant biasing member to undergo stress relaxation or creep.

Favorably the valve further comprises an inner seal and an outer seal. The inner seal is generally located relative to the canister towards the interior, while the outer seal is generally located further away from the interior. The outer seal is favorably located at or near the open end of the first valve body away from the interior. The inner seal is favorably located at or near the open end of the first valve body towards the interior.

Favorably the valve stem comprises a dispensing passage, wherein the valve stem is movable relative to the inner and outer seals, such that,
in the non-dispensing position of the valve stem, the dispensing passage is isolated from the metering chamber, and a communication path is provided between the aerosol container and the metering chamber, said path being either continuously or transiently open to allow substance to pass from the aerosol container to the metering chamber, and
in the dispensing position of the valve stem, said communication path between the aerosol container and the metering chamber is closed and the dispensing passage is in communication, either continuously or transiently, with the metering chamber to allow substance to be dispensed from the metering chamber through the dispensing passage.

Both the inner and outer seals may be provided as separate components, or alternatively to facilitate minimization of manufacturing costs and/or any or all performance issues outlined above, the first valve body and the inner seal or the outer seal or both seals may be advantageously integrally provided in a single component. This second integral component may be composed of a polymeric material, for example with one or more polymeric materials in a composite and/or including other materials such as fillers and/or reinforcing agents in one or more of the polymeric materials. This second component may be molded, in particular molded via one-shot molding or two or more shot molding.

In alternative embodiments including inner and outer seals, the inner seal may provided as an integral member of the integral single component comprising the second valve body and valve stem ("first integral component"), while the first valve body and outer seal are either provided separately or are provided integrally in a single second component.

Valves described herein desirably further comprise a ferrule for securely attaching the valve onto the aerosol container. The ferrule may be provided as a separate component or alternatively to facilitate minimization of overall number of components, the ferrule may be provided as an integral part of the second integral component.

Valves described herein desirably further comprise a gasket seal. The gasket seal generally facilitates sealing between the valve and the aerosol container when the valve is mounted onto the aerosol container. The gasket seal may be provided as a separate component, e.g. in the form of a rubber or elastomeric ring, or alternatively to facilitate minimization of overall number of components, the gasket seal may be provided as an integral part of the first integral component or as an integral part of the second integral component or as an integral part of the ferrule. Typically, the gasket seal is in the form of an annular ring with a generally rectangular cross-sectional profile.

Another aspect of the present invention is the provision of a canister comprising an aerosol container and a valve described herein, in particular a canister filled with an aerosol formulation, more particularly a medicinal aerosol formulation, even more particularly a pressurized, medicinal aerosol formulation, and most particularly a pressurized, medicinal aerosol formulation comprising medicament and a propellant, said propellant comprising HFA 134a and/or HFA 227.

Another aspect of the present invention is the provision of a medicinal delivery device comprising a valve described herein or a canister described herein. Advantageously the device is an inhalation device, in particular a pressurized medicinal inhalation device.

The above summary of the present invention is not intended to describe each disclosed embodiment nor every implementation of the present invention. The description that follows more particularly exemplifies illustrative embodiments. Also further embodiments are described in dependent claims. In several places throughout the application, guidance is provided through lists of examples, which examples can be used individually and in various combinations. In each instance, the recited list serves only as a representative group and should not be interpreted as an exclusive list.

### Brief Description of Drawing

The invention will now be described with reference to the accompanying drawings in which:
Figure 1 represents a cross-sectional illustration of a pressurized metered dose inhaler known in the art, while Figure 2 represents an enlarged partial view of the inhaler shown in Figure 1.

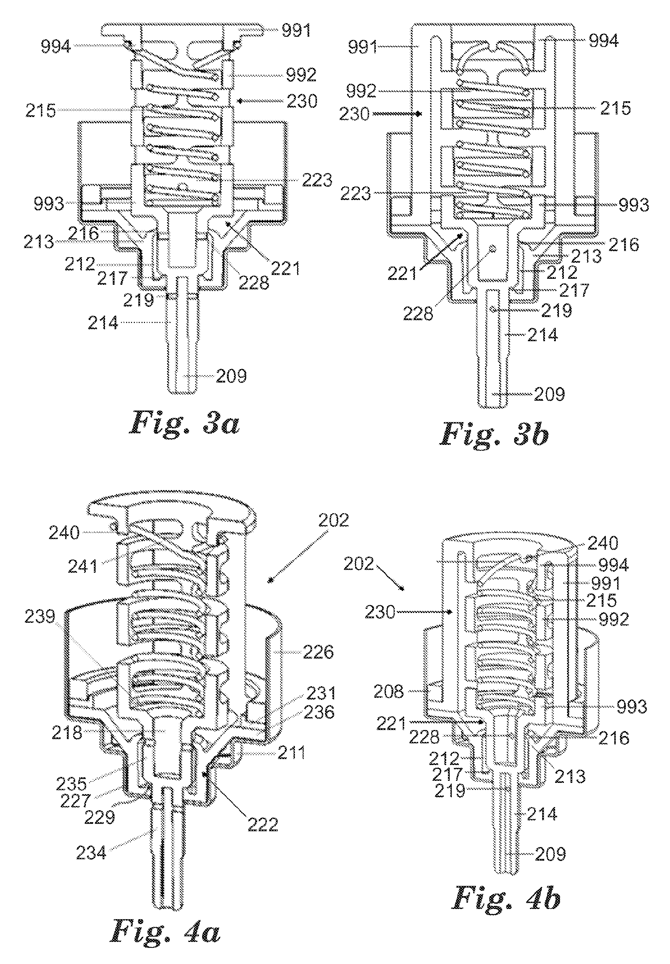

Figures 3a, 3b, 4a and 4b represent illustrations of an exemplary metered dose dispensing valve in accordance to the invention described herein, Figures 3a and 3b being cross-sectional lateral views of the valve sectioned in two mutually orthogonal vertical planes centrally through the valve, and Figures 4a and 4b providing the corresponding isometric views of the sectioned valve in the orientations shown in Figure 3a and Figure 3b, respectively.

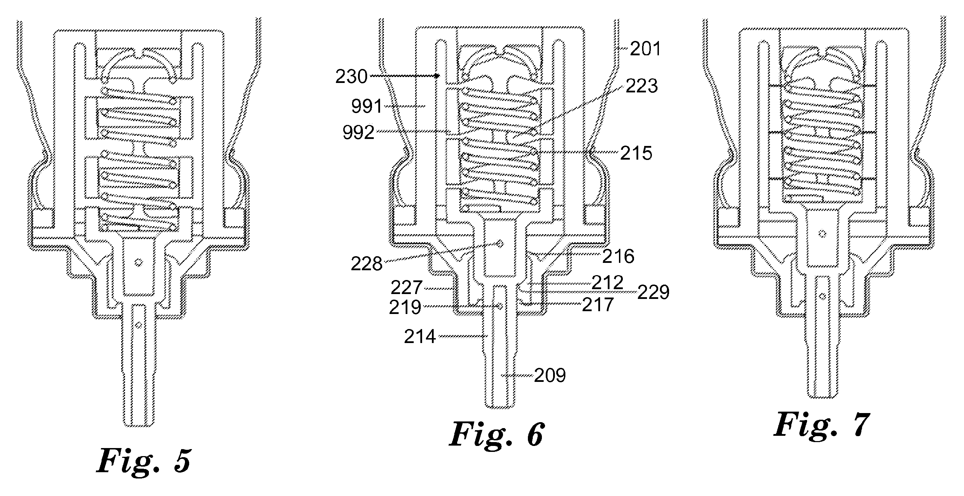

Figures 5, 6, and 7 represent cross-sectional lateral views of the exemplary valve shown in Figures 3 a,b and 4 a,b, where the valve is crimped to a container and shown in the orientation shown in Figure 3b and where Figure 5 (like Figure 3b) shows the valve in its rest position, Figure 7 its firing position, and Figure 6 a position therebetween.

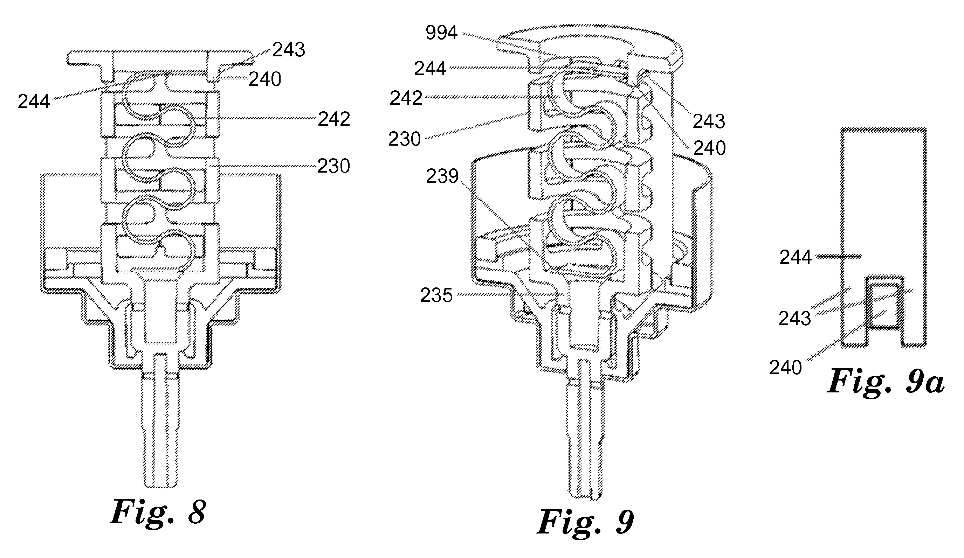

Figures 8 and 9 represent illustrations of another exemplary metered dose dispensing valve in accordance to the invention described herein, being a lateral view of a sectioned valve (Figure 8) and an isometric view of a sectioned valve (Figure 9), while Fig. 9a shows at a higher scale a horizontal cross-section of part of the valve.

In the description that follows, terms such as 'top', 'bottom', 'above', 'below', etc, refer only to features as shown in the Figures, and no restriction as to orientation of use, etc, is intended. Not all Figures are to the same scale.

### Detailed Description

It is to be understood that the present invention covers all combinations of particular, suitable, desirable, favorable, advantageous and preferred aspects of the invention described herein.

As discussed *sura,* Figure 1 and 2 show an exemplary, well known pressurized metered dose inhaler (Figure 1) or a detailed portion thereof (Figure 2). In particular, Figure 1 shows a metered dose canister (10) including an aerosol container (1) fitted with a metered dose valve (2) (shown in its resting position) as part of a metered dose dispenser (100), in particular an inhaler. The individual parts of the valve have been discussed *sura.* In operation, medicament formulation (4) can pass from the formulation chamber (3) into a pre-metering region (23) provided between the second valve body (20) housing and the first valve body (13) through an annular space (54) between a flange of the second valve body and the first valve body. To actuate (fire) the valve to deliver a dose of medicament formulation, the valve stem (14) is pushed inwardly relative to the aerosol container from its resting position shown in Figures 1 and 2, allowing formulation to pass from the metering chamber (12) through a side hole (19) in the valve stem (14) and through a stem outlet (24) out through an actuator nozzle (7) and then out to the patient. When the valve stem (14) is released, medicament formulation enters into the valve, in particular into the pre-metering chamber (23), through the annular space (54) and thence from the pre-metering chamber (23) through a groove (25) in the valve stem (14) past the inner seal (16) into the metering chamber (12). Because such valves retain the next dose of medication formulation in the metering chamber (12) between actuations, they are sometimes referred to as "retention valves". Retention valves represent the largest sector of the pMDI valve market.

Figures 3 to 9 provide illustrations of two exemplary embodiments of metered dose dispensing valves in accordance with the invention described herein. In these Figures, like reference numerals will denote similar or equivalent, but not necessarily identical, components or features.

Metered dose dispensing valves for dispensing metered volumes of a pressurized aerosol formulation from an aerosol container described herein comprise a first valve body defining in part a metering chamber and a valve stem passing axially through the metering chamber, movable relative to the chamber between non-dispensing and dispensing positions, and biased from its dispensing position towards its non-dispensing position by a compliant biasing member. The volume of the metering chamber (and thus the dose metered upon actuation) may favorably be within the range 25µl to 150µl, and more particularly 50µl to 65µl (end points inclusive for both named ranges). Desirably the first valve body has two open ends through which the valve stem passes, wherein relative to an affixed aerosol container, the first end is located towards the interior of the container and the second end away from the interior of the container. In use, the valve stem is moved axially against said bias from its non-dispensing position into its dispensing position and upon release the valve stem moves outwardly under the action of the bias from its dispensing position back to its non-dispensing position.

This can be better understood for example in reference to an exemplary embodiment shown in the Figures. Making reference to the views and exemplary embodiment shown in Figures 3 and 4, it can be seen that the embodiment includes a first valve body (213), defining a metering chamber (212), with inner and outer open ends. It also comprises a valve stem (214) passing axially through the openings in the first valve body. As will be described in more detail below in conjunction with Figures 5 to 7 *infa,* the valve stem (214) is movable relative to the chamber between non-dispensing and dispensing (firing) positions. The valve stem (214) is biased from its dispensing position towards its non-dispensing position by a compliant biasing member (215), desirably in the form of a spring member. As can be appreciated from the illustrations of this exemplary embodiment, the compliant biasing member (215) is provided as a compression spring member, more particularly a coil spring.

Metered dose dispensing valves for dispensing metered volumes of a pressurized aerosol formulation from an aerosol container described herein further comprise a second valve body defining at least in part a pre-metering region. The second valve body defines a space near the metering chamber to provide a pre-metering region such that the contents of the aerosol container will pass through the pre-metering region to the metering chamber, in particular the contents of the aerosol container will be fed directly and/or indirectly through the pre-metering region to the metering chamber. Advantageously the pre-metering region is located near the inner end of the metering chamber. The second valve body and valve stem are integrally provided in a single component.

Again by reference to the views and embodiment shown in Figures 3 and 4, it can be recognized that the exemplary valve includes a second valve body (230), defining a pre-metering region (223). The valve stem (214) is provided as part of the single integral component (221) together with the second valve body (230).

As mentioned above, metered dose dispensing valves for dispensing metered volumes of a pressurized aerosol formulation from an aerosol container described herein comprise a compliant biasing member that imparts a bias onto the valve stem biasing the valve stem towards its non-dispensing position. The compliant biasing member is positioned relative to the component comprising the valve stem and second valve body such that at least one portion of the biasing member engages said component and the at least one other portion of the biasing member engages elsewhere the said component such that it imparts a bias to the valve stem. Desirably the at least one portion of the biasing member engages the valve-stem-second-valve-body-comprising component such that the at least one portion of the biasing member is anchored relative to the valve-stem-second-valve-body-comprising component.

Favorably a portion of the second valve body provides a fixed support and/or anchoring position for the compliant biasing member. Moreover, the second valve body favorably comprises a non-compliant (e.g. rigid) portion, said portion being configured and arranged so that a fixed support and/or anchoring position is provided for the compliant biasing member, and a compliant (e.g. compressible) portion, this portion being configured and arranged so as to allow movement of the valve stem. Typically at least one part (e.g. one end or end region) of the compliant portion of the second valve body is joined either directly or indirectly to the valve stem and at least one other part (e.g. a second end or end region) is joined either directly or indirectly to the non-compliant portion of the second valve body. For such favorable embodiments, the at least one portion (e.g. one end portion) of the biasing member desirably engages the valve-stem-second-valve body component at a position proximate to or at the interface between the non-compliant and compliant portions of the second valve body towards the non-compliant portion and the at least one other portion (e.g. a second end portion) of the biasing member engages the valve-stem-second-valve body component at a position proximate to or at the interface between the compliant portion of the second valve body and the valve stem towards the valve stem. More desirably for such embodiments the at least one portion (e.g. one end portion) of the biasing member engages the non-compliant portion of the second valve body at a position proximate to or adjacent to the interface between the non-compliant and compliant portions of the second valve body and the at least one other portion (e.g. a second end portion) of the biasing member engages the valve stem at a position proximate to or adjacent to the interface between the compliant portion of the second valve body and the valve stem.

Referring to views and exemplary embodiment shown in Figures 3 and 4, it can be recognized that the second valve body (230) of the exemplary valve includes a non-compliant portion (991) and a compliant portion (992), where one end (993) of the compliant portion connects to the valve stem (214) and the other end (994) connects to the non-compliant portion.

As can be appreciated from the exemplary embodiment, the second valve body preferably has a cage-like form. A cage-like form for the second valve body is advantageous in that such form is discontinuous and allows for large gaps and ready access of medicament formulation to the internal pre-metering region defined by such a body as well as facilitating injection molding.

Also as can be appreciated from the exemplary embodiment, for those favorable embodiments where second valve body includes a non-compliant and a compliant portion, desirably the portions are nested. Moreover desirably one portion (e.g. the compliant portion) is fully or partially contained within the other portion (e.g. non-compliant portion) of the second valve body. In the exemplary embodiment, it can be seen that the two portions (991,992) of the second valve body form a cage-in-cage-like structure with compliant portion (992) being nested in the non-compliant portion (991).

Favorably, the single integral component comprising the second valve body and the valve stem may comprise a polymeric material, for example either a single polymeric material or with one or more polymeric materials in a composite and/or including other materials such as fillers and/or reinforcing agents in one or more of the polymeric materials. Such components may be favorably molded, in particular via one-shot molding or two or more shot moldings. Favorably, the polymeric material or materials selected will have adequate strength, stiffness and toughness to form suitable compliant and non-compliant portions. Advantageously the compliant portion of the second valve body will employ a material or materials of adequate Young's Modulus to ensure at least that the different parts and portions of the second valve body are held relative to one another in positions well enough defined for automated handling and assembly systems and operations. Typically, the non-compliant portion of the second valve body will employ a material or materials of adequate stiffness to resist the biasing forces experienced during use of the assembled valve without excessive deformation, creep or risk of fracture or other failure mode. Advantageously, a single polymeric material with properties suitable simultaneously for both the compliant and non-compliant portions of the single integral component comprising the second valve body and the valve stem will be selected. Examples of suitable polymers for use to make such integral components include acetal (polyoxymethylene) polymers (such as Delrin^{TM} (Dupont de Nemours & Company)), polyetherimides (e.g. ULTEM 1000), liquid crystalline polymers (LCP) or polyetheretherketones (PEEK). Other examples of suitable polymeric materials exhibiting low levels of creep include polyvinylidene difluoride (PVDF), polycarbonate, polyethersulphone (PES) and phenolic laminates. Preferably, food-grade or pharmaceutical-grade materials would be used, although standard industrial grades could be used for non-pharmaceutical applications. Suitable fillers and/or reinforcing agents include fibers, such as glass fibers or carbon fibers.

Valves described herein are favorably configured and arranged such that, in use, when the valve stem is in its non-dispensing position, the pre-metering region is in communication either continuously or transiently with the contents of the aerosol container and the metering chamber is in communication at least transiently with the pre-metering region to allow substance to pass from the aerosol container to the metering chamber, and when the valve stem is in its dispensing position, the pre-metering region is isolated from the metering chamber and a communication path is provided between the metering chamber and the outside of the valve and aerosol container assembly, said path being either continuously or transiently open to allow substance to pass from the metering chamber to the outside.

Once again by reference to the views and embodiment shown in Figure 3 and 4, it can be recognized that the exemplary valve includes a compliant biasing member (215) is in the form of a coil spring. As shown in the exemplary embodiment, the biasing member may be favorably located at least in part in the pre-metering region (223). This can be advantageous in that trapping the biasing member within the pre-metering region facilitates minimization of tendencies of the biasing member towards lateral relative movement or separation from the valve during use. The coil spring (215) has a narrow end and a wide end, the wide end having an elliptically elongated coil (241) which is 'v' shaped when viewed across the longer part of the ellipse, i.e. its profile is like an ellipse kinked about the minor axis, and the spring wire ends just short of where its path would complete the bent ellipse, with the 'v' diverging towards the wide end of the coil spring. To assemble the coil spring, the narrow end is inserted into a hole at the top end of the second valve body (230) until it bottoms out on the ledge (239) at the top of the inner stem part (235). Downward compression of the coil spring causes the wide end to slip through the hole, bending the ellipse further as it goes. Further compression allows the elliptically elongated coil (241) to emerge through spaces at the top end (994) of the compliant portion of the second valve body (230), and for the widest parts to wrap around lugs (240) downwardly depending from the top end (994) of the second valve body (230), in order to lock it in place. Thus after assembly one end of the compliant biasing member, i.e. the wide end, engages and is anchored onto the non-compliant portion (991) of the second valve body (230) near the interface between the non-compliant portion and compliant portion (992) of the second valve body, while the other end, i.e. the narrow end, of the compliant biasing member engages the valve stem (214), i.e. engages the top end of the valve stem, near the interface between the valve stem and the compliant portion of the second valve body.

Advantageously compliant biasing members are spring members. Depending on the particular valve design, compliant biasing members may be compression or tension spring members. It is well known that compression springs become shortened (e.g. are compressed) when pressure is applied to them, generally offering resistance to a compressive force applied axially, while tension springs become extended (e.g. are stretched) when pressure is applied, generally offering resistance to an extensive force applied axially. Such spring members may have an overall cylindrical, frusto-conical, hourglass (convex), or barrel (concave) shape. They may have a conventional coil or helical, leaf, or stack spring configuration. Commercial pressured metered dose inhalers generally comprise a helical metal coil, cylindrical, compression spring, although other types of spring are known to be applied to aerosol valves in the patent literature, e.g. EP 1477234 and EP 1565270 both mention a stack spring for metering valves. Compliant biasing members may alternatively have less conventional spring configurations, such as C-springs.

Compliant biasing members may comprise metal. Compliant biasing members may comprise a polymeric material, for example either a single polymeric material or with one or more polymeric materials in a composite and/or including other materials such as fillers and/or reinforcing agents in one or more of the polymeric materials. Such components may be favorably molded, in particular via one-shot molding or two or more shot moldings. Alternatively compliant biasing members may favorably comprise metal and a polymeric material, for example when the compliant biasing member is made of metal and then coated with a (e.g. low energy) polymeric coating or when the compliant biasing member includes a polymeric structure reinforced with a metallic sub-structure. The latter may be desirably molded by insert molding in conjunction with one-shot or two or more shot polymeric molding (e.g. insert a metal spring into the appropriate portion of the component mold and then mold, if applicable overmold, the polymeric elements of the component). Advantageously, the compliant biasing member comprises a metal without a polymeric material or materials. Alternatively, where the compliant biasing member comprises a polymeric material without a metallic sub-structure of any kind, typically the polymeric material or materials selected will have adequate strength, stiffness and toughness to provide the appropriate bias, without excessive deformation, creep or risk of fracture or other failure mode. Examples of suitable polymers for use in making polymeric compliant biasing members include acetal (polyoxymethylene) polymers (such as Delrin^{TM} (Dupont de Nemours & Company)), polyetherimides (e.g. ULTEM 1000), liquid crystalline polymers (LCP) or polyetheretherketones (PEEK) as well as mixtures and composites thereof. Other examples of suitable polymeric materials exhibiting low levels of creep include polyvinylidene difluoride (PVDF), polycarbonate, polyethersulphone (PES) and phenolic laminates. Again it would be preferable to use food-grade or pharmaceutical-grade materials, although standard industrial grades could be used for non-pharmaceutical applications. Again suitable fillers and/or reinforcing agents include fibers, such as glass fibers or carbon fibers. In the event that the compliant biasing member is made of metal per se (either non-coated or coated) or includes a polymer-based over-structure reinforced with a metallic sub-structure, suitable metals include stainless steel. Where the compliant biasing member incorporates a polymer as well as a metal, either as a polymer-based structure reinforced with a metallic sub-structure or as a coating on a metal component or sub-structure, the restrictions on the choice of polymeric material or materials may be less onerous, as much or all of the bias may be provided by the flexural properties of the metal component or sub-structure. In such a case, almost any polymeric material or materials could be employed, with polymeric materials selection being based on reasons of surface energy (e.g. low surface energy, for low drug particle deposition), surface chemistry (e.g. for low reactivity with drug formulation constituents), or some other reason rather than mechanical and flexural properties. Suitable additional polymer classes include polyolefins, fluoropolymers (such as olefinic fluoropolymers (including perfluoroalkoxy polymers), perfluoroethers, fluoroacrylates, fluorosilicones, perfluoropolyether silanes, perfluoropolyether phosphates, fluoroalkylsilanes, fluoroparylenes), polymerized siloxanes (e.g. dimethylsiloxane, diphenylsiloxane, hexamethyldisiloxane, tetramethyldisiloxane), silazane polymers, alkoxysilane polymers, Parylene N, Parylene C, and Parylene D.

As stated *supra,* valves described herein favorably include an inner seal and an outer seal. Generally the outer seal is desirably located at or near the open end of the first valve body away from the interior, while the inner seal is favorably located at or near the open end of the first valve body towards the interior. The outer seal may be in sliding sealing engagement with the valve stem. Preferably the inner seal is in the form of a lip seal, which allows formation of a relatively low friction sliding seal with the valve stem component during valve operation. Such a seal is partially pressure-assisted during valve firing, in that the pressure difference that develops across it as the metering chamber empties helps to maintain a good seal while the stem (214) remains pushed in towards the aerosol container. Use of a lip seal type of inner seal also facilitates easy pressure-filling of the system. (Pressure filling, or previous air-blowing, can be used to ensure that the inner seal is in its correct as-shown orientation, e.g. if valve assembly turns it 'inside out'.)

Valve stems typically comprise a dispensing passage, and desirably are movable relative to the inner and outer seals, such that, in use,
in the non-dispensing position of the valve stem the dispensing passage is isolated from the metering chamber, and a communication path is provided between the aerosol container and the metering chamber, said path being either continuously or transiently open to allow substance to pass from the aerosol container to the metering chamber, and
in the dispensing position of the valve stem said communication path between the aerosol container and the metering chamber is isolated and the dispensing passage is in communication, either continuously or transiently, with the metering chamber to allow substance to be dispensed from the metering chamber through the dispensing passage.

Again referring to the views and embodiment in Figures 3 and 4, it can be seen that the valve stem (214) includes a dispensing passage (209), and at the inner end of the metering chamber there is an inner seal (216) in the form of a lip seal, while at the opposite, outer end of the metering chamber there is an outer seal (217). In this embodiment, both seals are in non-transient, sliding engagement with the valve stem (214). Near the closed end of the dispensing passage is a side hole (219), and in the inner bore of the inner portion of the valve stem is another side hole (228). The two side holes are termed the dispensing side hole and filling side hole, respectively.

Reference is now made to Figures 5 to 7, showing the exemplary valve illustrated in Figures 3 and 4 crimped to an aerosol container, to explain the positions of the valve stem in operation. To ease in viewing and comparison of the positioning, only Figure 6 has been marked with reference numbers. First looking from Figure 5 showing the valve in its rest position to Figure 7 showing the valve at its dispensing (firing) position, it will be noted that the valve stem has moved inwardly towards the aerosol container (201). Moreover, the exemplary valve is similar to industry-standard push-to-actuate pMDI valves in that, in use, the valve stem is moved axially, e.g. by the user or a breath actuated mechanism, inwardly towards the aerosol container. As mentioned previously, the compliant biasing member (215) is biasing (outwardly away from the aerosol container) the valve stem (214) from its dispensing position towards its rest position. At the rest position of this exemplary valve, the dispensing passage (209) is isolated from the metering chamber since the dispensing side hole (219) is on the outside of the canister at this position. Also there is a communication path between the aerosol container (201) and the metering chamber (212) because there is a communication path from the metering chamber via the filling side hole (228) through the upper stem portion of the valve stem and the pre-metering region (223). When the user starts to actuate the valve by causing the valve stem to push inwardly, the filling side hole (228) and the dispensing side hole (219) start to pass by the inner seal (216) and outer seal (217), respectively. Figure 6 shows a position between the rest position and dispensing position where the metering chamber is completely isolated. Specifically, the metering chamber is isolated from the pre-metering region, since the filling side hole (228) is no longer located in the metering chamber, and the metering chamber is (still) isolated from the dispensing passage (209) since the dispensing side hole (219) is not (yet) located in the metering chamber. As the user continues to actuate and as the dispensing side hole (219) completely passes the outer seal, the dispensing passage (209) of the valve stem (214) is in communication with the metering chamber to allow substance to be dispensed from the metering chamber through the dispensing passage (Figure 7). This is a result of the dispensing side hole (219) being now located within the metering chamber (212). Looking from Figure 5 to Figure 7, from the valve's rest position to its firing position, it will be noted that both the compliant biasing member (i.e. the coil spring) and the compliant portion (992) of the second valve body (230) have been compressed. Also it will be noted that the non-compliant portion (991) of the second valve body (230) remains fixed during operation. The end point of the inward movement of the valve stem is provided by the design of the compliant portion of the second valve body. In particular, this is achieved by designing the compliant portion of the second valve body in the form of a stack-spring and configuring it such that the different arms of its stack-spring design come to rest solidly against one another, preventing further deformation of the compliant portion of the second valve body and thus limiting travel of the valve stem. After firing and as the user lets the valve return towards its rest position, the valve stem (214) moves outwardly (away from the interior of the aerosol container (201)) under the biasing action of the compliant biasing member (215) and, if applicable, under the additional biasing action of the compliant portion of the second valve body, and the filling side hole (228) enters the now empty metering chamber (212) establishing once again a communication path between the metering chamber and the interior of the aerosol container, thus allowing substance to pass from the aerosol container to the metering chamber.

Valves described herein may advantageously include the first valve body and the inner seal or the outer seal or both seals integrally provided in a single integral component ("second integral component") to yet further minimize the overall number of components. Alternatively for valves using an inner seal that operates as a face seal, the inner seal may be provided as an integral member of the component comprising the valve stem and second valve body ("first integral component"), while the first valve body and outer seal may be either provided separately or be provided integrally in a single second component.

In the exemplary embodiment shown in Figures 3 to 7, both the inner and outer seals (216,217) are provided together with the first valve body (213) in an integral component (222). This integral component combines at least three separate functions that are conventionally each provided by separate components. The first two of these functions are inner and outer seals, as will be apparent by analogy with the valve of Figures 1 and 2, whilst the third function is the definition of a metering chamber (212).

Integral components comprising the first valve body and the inner seal and/or the outer seal are favorably composed of a polymeric material. Such integral components may be molded, in particular molded via one-shot molding or two or more shot molding. It will be noted that such integral components (e.g. that of the embodiment of Figures 3 to 7) may require 'bumping' off the molding tool (i.e. the molded components may be required to transiently elastically distort as they are ejected from the mold cavity of the injection molding tool), due to the undercuts in the design (e.g. the undercut of the inner seal (216)). This 'bump off process may be air-assisted. Such components may be formed from readily moldable thermoplastic elastomer (TPE) material or thermoplastic vulcanizate (TPV) material. Preferably, food-grade or pharmaceutical grade materials would be used, although standard industrial grades could be used for non-pharmaceutical applications. Examples of commercial TPE materials are copolymers ofbutylene terephthalate and polyalkylene ether terephthalate, such as HYTREL SC938 TPE or HYTEL SC948 TPE from Dupont de Nemours & Company, the former material having a Shore D hardness of 30, the latter a Shore D hardness of 40. Examples of suitable TPV materials are polyolefinic dynamic vulcanisates of rubber particles in a thermoplastic, such as SANTOPRENE (Exxon Mobil Corporation), e.g. grade 121-62M100, ester-based thermoplastic polyurethanes, such as ESTANE™, polyether polyamide block copolymers, such as PEBAX™, polyolefin elastomers, such as ENGAGE™, polyethylenebutylenes, such as FLEXOMER™ GERS 1085NT, ethylene alpha olefin copolymers, such as EXACT™, or polymers with included Exxelor™ polymer resins. Other materials include co-vulcanisates of ethylenevinylacetate with butyl rubber or a halobutyl rubber and a rubber selected from Neoprene, ethylene propylene diene monomer (EPDM), ethylene propylene copolymer rubber (EPR) and polypropyleneoctene, as disclosed in WO2003/18432. Other materials include those disclosed in GB2410500 (seals comprising 1. elastomeric alkene 2. thermoplastic 3. sensitisor eg acrylate), WO2006/092618 (TPE seal material having propylene units with isotactic crystalline form), and GB2323597 (contains TPE, but may not itself be TPE). Alternatively, the components may be composed of rubber materials such as EPDM, Neoprene, Nitrile, Butyl or Chlorobutyl rubber. Surprisingly, such materials can suitably provide the requisite combination of sealing (and low friction sliding seal surfaces) and structural rigidity (and controlled swell in the formulation (4)) for definition of a metering chamber of known volume (and hence of known metered dose size) in a molding of suitable design (such as those of the exemplary designs shown in Figures 3 to 9). If desired and/or needed, the polymeric material may be a composite including e.g. fillers and/or reinforcing agents. Advantageously, the polymeric material would have a Shore D hardness of between 25 and 55, more advantageously between 30 and 50 inclusive.

Valves described herein may include a ferrule. Ferrules are typically made by conventional deep-drawing from aluminum alloy strip metal, although alternative forms, such as injection molded plastics, could be used. In a deep-drawn form, the ferrule may comprise a skirt region for attachment onto the neck of the aerosol container by a conventional multi-jaw crimping process. Other processes could however be employed to affix and seal the ferrule to the aerosol container, for example rotary crimping, laser welding or ultrasonic welding. Alternatively, a screw-on attachment could be used, e.g. for an injection molded plastic ferrule.

Valves described herein may further comprise a gasket seal. Such a gasket seal may be provided separately, or once again to aid minimization of the overall number of components it may be provided either as an integral part of the second component, or as an integral part of the first component, or as an integral part of the ferrule.

For valves including a ferrule and a component comprising integrally the first valve body defining at least part of the metering chamber together with the inner seal and/or the outer seal, it is advantageous to size with care said integral component relative to the ferrule due to the intrinsic partially flexible nature of the integral component required for its sealing function. Also, it is advantageous to provide a close radial fit between part of (the inner wall of) the ferrule and part or all of the first valve body's radially outer wall, and to facilitate support of the first valve body in order to help ensure that it cannot flex radially outwardly enough to adversely affect control of the volume of the metering chamber. Accordingly, in such advantageous embodiments, the ferrule is formed such that a portion thereof defines an alcove-space and at least a portion (preferably a substantial portion) of the first valve body is located within the alcove-space such that at least a portion (preferably a substantial portion) of the radially outer wall of the first valve body is adjacent to the inner wall of the portion of the ferrule defining the alcove-space.

To help ensure a complete understanding of the exemplary embodiment shown in Figure 3 to Figure 7, reference is made to the views shown in Figures 3 and 4. It can be appreciated that the exemplary embodiment of the metered dose dispensing valve includes a total of five components, i.e. the first integral component (221), the second integral component (222), the compliant biasing member (215), the ferrule (211) and the gasket seal (208): about a 40% reduction in the number of components of the commercial valve shown in Figure 1. For ease in discussion, in the following, the first integral component including the second valve body and the valve stem will be called in the following the antechamber component, and the second integral component including the first valve body together with both the inner and outer seals will be called the metering chamber component.

The antechamber component (221) of the exemplary valve (202) provides multiple integral features and elements. In particular, it provides a valve stem (214) including a hollow male stem part (234) protruding through a piercing in the ferrule (211) and an inner stem part (235) having an internal stem passage (218) and a second valve body (230) defining a cage-in-cage-like structure enclosing a pre-metering region (223) and having a gasket rim (231) that gets fixedly located by the valve to vial crimping operation. The cage-in-cage-like structure is discontinuous, with large gaps to allow ready access of medicament formulation to the inside regions (and to facilitate injection molding).

An internal passage (218) in the inner stem part (235) leads from the interior of the pre-metering region (223) to the metering chamber (212) via the filling side hole (228). The stem dispensing side hole (219) leads from the metering chamber (212) to the dispensing passage (209) provided as a bore of the protruding hollow male stem part (234) when the stem component (214) is pushed in towards the aerosol container (201). A step on the radially outer surface of the valve stem provides an axial stop (229): this comes into contact with the outer seal (217) when the stem component (214) is pushed outwardly away from the aerosol container (201) by a combination of the compliant biasing member (215), which is provided in the form of a metal coil spring, the medicament formulation vapor pressure, and, if applicable, the complaint part of the second valve body (962), thereby providing both a defined rest position for the valve stem and also an axial seal to minimize formulation leakage during long-term storage. In addition, the valve (202) is preferably designed so that the compliant biasing member (215) provides a measure of outward bias to the valve stem (214), in particular onto the inner stem part (235), when the valve is in its rest position, thus helping to ensure both complete stem return and also good at-rest axial sealing. This outward bias is of course additional to any (temperature-dependent) biasing force provided by the formulation's vapor pressure. It is also worth noting that any temperature dependence of the compliant biasing member (215), for example if it is formed from a polymeric material that appreciably reduces in stiffness as the temperature rises, will tend to be offset by this vapor pressure biasing force tending to increase with rising temperature.

The metering chamber component (222) of the exemplary valve (202) provides multiple integral features and elements, such as a first valve body (213) defining at least in part the metering chamber plus the inner seal (216) and outer seal (217). The component also includes an integral, radially outwardly extending shelf member (236) in the form of a ring (closed or open, i.e. continuous or discontinuous) connected to the radially outer wall of the first valve body. This shelf member may desirably facilitate placement of the antechamber component and/or support of the metering chamber component within the ferrule (in particular near the shoulder of the ferrule) during assembly of the valve. For example, the shelf member can be dimensioned such as to abut the inner walls of the ferrule and thereby to align the antechamber component centrally within the ferrule. Use of a slight dimensional interference fit between the shelf member and the inner walls of the ferrule can serve to align and retain both the antechamber component and the metering chamber component within the ferrule prior to subsequent crimping onto an aerosol container.

The ferrule (211) of the exemplary valve (202) includes a ferrule skirt (226) allowing for crimping onto the aerosol container (201; see Figures 5 to 7). A portion of the ferrule (227), in particular the central portion thereof, forms an alcove-space. The ferrule is provided with an opening, typically a piercing, to allow the hollow male stem part (234) to pass through.

To assemble the exemplary valve (202) shown in Figures 3 to 7, the hollow male stem part (234) of the valve stem (214) is pushed through the inner (216) and outer seals (217) of the metering chamber component (222) and the gasket rim (231) of the antechamber component (221) is pushed against the upper face of the radially outer portion of the shelf member (236) of the metering chamber component. Thereafter the hollow male stem part (234) is inserted through the ferrule piercing, with simultaneous placement of a substantial portion of the first valve body (213) into the alcove-space of the ferrule and placement of the lower face of the radially outer region of the shelf member onto the inside of a shoulder of the ferrule. Thereafter the gasket (208) is placed onto the gasket rim (231) of the antechamber component (221), Finally, the compliant biasing member is inserted as described above, the valve now being ready for aerosol container (201) crimping and filling. It will be appreciated that the insertion of the compliant biasing member into the pre-metering region of the antechamber component can be done prior to any of the assembly steps described above. If desired, small nibs in the ferrule's inner walls, and/or a slight crimp, could be used to supplement a dimensional interference-fit as a means of holding the assembled valve together prior to crimping it onto an aerosol container. (Although not shown in the exemplary embodiments, if desired an O-ring like that shown in Figures 1 and 2 (see element with reference number 53) could be placed around the narrow portion of the aerosol container between the aerosol container and the ferrule skirt prior to crimping. Use of such an O-ring could be either in addition to use of a gasket (208) or could be instead of use of such a gasket.)

Medicament aerosol formulation would be filled into the aerosol container (201) either before (cold-filling) or after (pressure filling) affixing the valve onto the aerosol container. With only five (or six, when counting an additional O-ring) components to assemble, rather than the eight (or nine, when counting an additional O-ring) of many commercially available valves, the assembly process can be simpler, quicker and cheaper than current pMDI valve assembly processes. In use, referring to Figures 5 to 7 and the description above, the exemplary valve operates in an analogous manner to the prior art conventional retention valve of Figures 1 and 2.

Figures 8 and 9 illustrate a second exemplary valve in accordance to the present invention. This exemplary embodiment is analogous to the exemplary embodiment depicted in Figures 3 and 7 with the exception that the compliant biasing member is provided in the form of a metal compression spring in the form of a leaf spring (242). The leaf spring is made as a metal strip bent into an undulating shape along a longitudinal direction except for the ends which are flat, orientated parallel to each other and perpendicular to the longitudinal direction, and pointing in opposite directions. At one end (244) of the spring, the metal bar extends beyond the lateral envelope of the undulations and is provided with a forked portion having prongs (243).To assemble the leaf spring, the non-extended end is inserted into a hole at the top end of the second valve body (230) until it bottoms out on the ledge (239) at the top of the inner stem part (235). Downward compression of the leaf spring causes the extended end (244) to slip through the hole, bending it diagonally upwards as it goes. Further compression allows the extended end (244) to emerge through spaces at the top end (994) of the compliant portion of the second valve body (230), and for the fork to surround a lug (240) downwardly depending from the top end (994) of the second valve body (230), in order to lock it in place. Figure 9a shows at a higher scale a horizontal cross-section through the extended end (244) of the leaf spring (242), and that the lug (240) lies between the prongs (243) of the fork portion.

Taken together, the Figures and the illustrated exemplary embodiments show that metering valves in accordance to the invention are unique valves, suitable for use in pMDIs, that show simplicity and low component count (e.g. five components in these exemplary valve embodiments, versus typically eight components in many commercially available pMDI valves), whilst providing familiar conventional axial push-to-fire operation and desirably with conventional interfacing to the actuator (via a hollow male valve stem) and to the aerosol container (via a conventional crimp).

To aid in avoiding deposition of medicament and/or to enhance frictional properties for smooth operation, a part or all of the interior surfaces of the metering valve (e.g. part or all of the surfaces of the antechamber component and/or the metering chamber component and/or compliant biasing member) may be provided with a low energy surface coating. Examples of such coatings include plasma coatings such as DLG (diamond-like glass) as disclosed in WO2009/061895 and WO2010/129753 and perfluoropolyether silane coatings optionally superimposed on a non-metallic, e.g. DLG, base coating as disclosed in WO2009/061891, WO2009/061907, WO2009/061902 and WO2010/129758. Other possible coatings include plasma polymerized fluorinated hydrocarbons, chemical or physical vapour deposited polymers, cold plasma polymerized siloxanes, e.g. dimethylsiloxane, diphenylsiloxane, hexamethyldisiloxane, tetramethyldisiloxane, silazanes, alkoxysilanes, Parylene N, fluoroparylene, Parylene C, Parylene D, fluoroacrylates, coatings of perfluoropolyethersilane, perfluoropolyether phosphate and/or fluoroalkylsilane, where such coatings are deposited either by dipping, spraying or pouring, and causing or allowing the molecular attachment groups to cure, or by plasma deposition, vacuum deposited silica (about 500 nm thick) on steel component surfaces by a process known as the Silcosteel® process, and fluoroalkyl monolayer coatings as described in WO2007/112312.

As mentioned above, it is desirable to provide valves that can interface in a conventional way to typical aerosol containers used in pressurized metered dose inhalers. Such aerosol containers are typically made of a metal (e.g. aluminum or aluminum alloy or stainless steel). In such cases, typically it is advantageous to use mechanically crimpable ferrules (e.g. ferrules made of metal, such as aluminum or aluminum alloy). Aerosol containers may be made of other materials, such as glass, plastic or ceramics. Aerosol containers may be coated and/or the interior surfaces of the metering valve may be coated on part or all of their interior walls to reduce drug deposition, e.g. with any of the coatings listed in the previous paragraph. Alternatively, a coating may be selected from mixed fluoropolymer and nonfluoropolymer, where the fluoropolymer is e.g. polytetrafluoroethylene (PTFE), copolymerized ethylene tetrafluorethylene (ETFE), copolymerized perfluoroethylene propylene (FEP), perfluorinated polyalkoxyethylene-co-ethylene (PFA), polyvinylidene difluoride (PVDF), polymerized chlorinated ethylene tetrafluoroethylene (CETFE), and the non-fluoropolymer is e.g. a polymer selected from the following families of polymers: polyethersulphone (PES), polyamideimide (PAI), polyphenylenesulphide (PPS), polyamide, amine-formaldehyde thermosetting resin, benzoguanamine and/or polyethyleneglycol (PEG). Preferred options are PTFE-PES, FEP-PES and PFA-PEG. Aluminum aerosol containers may be anodized, and the anodized surface may help other coatings like PTFE or PFA to adhere more firmly to the container. The aerosol container may be coated with a fluoropolymer by electrostatic dry powder coating. Other coatings may include epoxy-phenolic or epoxyurea-formaldehyde linings (e.g. the epoxy/phenol formaldehyde resins described in WO95/17195).

In the event that a plastic aerosol container (e.g. a blow-molded or injection molded plastic container) is used rather than a conventional metal aerosol container, it may be desirable to use a plastic ferrule (e.g. an injection-molded ferrule) instead of a metal one. Here the plastic ferrule and aerosol container may be designed to allow the ferrule to clip or screw onto the aerosol container, or be equipped with co-operating surfaces for ultrasonic, laser or other thermal welding of the ferrule to the aerosol container. Alternatively, adhesives might be used to affix the valve onto the aerosol container.

It may be desirable to provide the ferrule as an integral element of the metering chamber component, to advantageously yet further reduce the number of components. The manufacture of such integral components may include single-shot molding or more advantageously at least two-shot molding, e.g. overmolding the other element(s) of the metering chamber component into a formed ferrule or alternatively overmolding a ferrule onto the other formed element(s) of the metering chamber component. It will be appreciated that by providing the ferrule (and the optional gasket seal) as an integral element(s) of the metering chamber component may allow for the provision of a three-component axial metering valve design.

In the previous exemplary embodiments the compliant biasing member is located at least in part (in particular, completely) within the pre-metering region. This is advantageous in that it allows for the production of compact valves with their compliant biasing members protected within their second valve bodies. The latter can be useful in terms of manufacturing, handling and transport of valves and/or individual components thereof. Nonetheless alternative designs are possible where the compliant biasing member may be located in part or completely outside the pre-metering region.

Although not illustrated, it will be appreciated that once a metering valve of one of the types described herein is affixed to an aerosol container, a valve and aerosol container assembly (a "canister") is provided such that the inner walls of the aerosol container and the outer envelope of the metered dose valve located within the aerosol container define a formulation chamber in which medicinal aerosol formulation may be contained.

Canisters fitted with a metering valve described herein may be advantageously utilized as part of dispensers for the administration of medicament through oral, nasal, transmucosal (e.g. buccal, sublingual), vaginal, rectal, ocular or aural delivery. Canisters fitted with a metering valve described herein are particularly suited for delivering medicaments by inhalation to a patient. Accordingly, metering valves described herein and canisters fitted with such valves are particularly suitable for use in or as pressurized metered dose inhalers, respectively.

As indicated above, desirably valves described herein are used in standard pressurized metered dose inhalers, and thus it is favorable that the valves have appropriately dimensioned features to interface with the neck of a standard container (although atypical containers could be provided at the expense of new deep drawing tooling and new feed lines and transfer housings for the container making machine). The neck of a typical container has an opening of about 17 mm diameter comprising a rim and a neck, with a bead between the rim and the neck. Consequently, it would be favorable to provide valves such that the widest part of the valve that would be inserted into the container upon mounting the valve on the container to form a canister (in other words the widest insertable width) would be less than 17 mm in diameter. A gasket is typically included in the valve to seal against the rim, although as illustrated above other means for attachment may be used while maintaining the same outer profile of inhaler unit for use in typical actuators. The depth of the smallest, presently commercially used can is about 28 mm from rim to recessed base. Consequently, it would be favorable to provide valves such that the longest part of the valve that would be inserted into the container upon mounting the valve on the container to form a canister (in other words the insertable depth of the valve) would be less than 28 mm.

Medicinal aerosol formulations may include any drug or combination of drugs that can be delivered by an aerosol (e.g. administered by inhalation) and such drug or drugs can be provided in suspension and/or solution in liquefied propellant, in particular liquefied HFA 134a and/or HFA 227. If desired or deemed necessary, medicinal aerosol formulations may comprise one or more other non-HFA 134a/HFA 227-propellant components, such as excipients, surfactants and suspending aids.

For manufacture of medicinal aerosol canisters filled with a formulation of drug or drugs in suspension, particulate drug in dry powder form may be and is often supplied in micronized form from the producer of the active ingredient. Micronization can be accomplished, e.g., by using a fluid energy mill driven by compressed air, such as shown in 'Drug Delivery to the Respiratory Tract' ed. D.Ganderton and T.Jones, publ. Ellis Horwood, Chichester (1987) pages 89-90, or by repeated stepwise millings or by use of a closed loop milling system.

The primary particle size of drug (e.g. the size upon completion of micronization) generally has a mass median particle diameter of 5 microns or less, and most suitably said mass median diameter is in the range 0.8 to 3 microns, with at least 90 % by mass of the particles having diameters below 5 microns, which can be determined, for example, by using an Andersen Cascade Impactor.

Depending on the particular valve and/or filling system used, aerosol formulation may be filled into the aerosol container either by cold-filling (in which chilled formulation is filled into the aerosol container and subsequently the valve is fitted onto the aerosol container) or by pressure filling (in which the valve is fitted onto the aerosol container and then formulation is pressure filled through the valve into the aerosol container).

Suitable drugs include those for the treatment of respiratory disorders, e.g., bronchodilators, anti-inflammatories (e.g. corticosteroids), anti-allergics, anti-asthmatics, anti-histamines, and anti-cholinergic agents. Other drugs such as anorectics, anti-depressants, antihypertensive agents, anti-neoplastic agents, anti-tussives, anti-anginals, anti-infectives (e.g. antibacterials, antibiotics, anti-virals), anti-migraine drugs, anti-peptics, dopaminergic agents, analgesics, beta-adrenergic blocking agents, cardiovascular drugs, hypoglaecemics, immunomodulators, lung surfactants, prostaglandins, sympathomimetics, tranquilizers, steroids, vitamins, sex hormones, vaccines, therapeutic sense or anti-sense nucleic acids, and other therapeutic proteins and therapeutic peptides may also be employed for delivery by inhalation.

Exemplary drugs which may be employed for delivery by inhalation include but are not limited to: albuterol, terbutaline, pirbuterol, fenoterol, metaproterenol, isoproterenol, isoetharine, bitolterol, epinephrine, tulobuterol, bambuterol, reproterol, adrenaline, ipratropium, oxitropium, tiotropium, darotropium, glycopyrronium, aclidinium, umeclidinium, troventol, beclomethasone, betamethasone, flunisolide, budesonide, mometasone, ciclesonide, rofleponide, aminophylline, dyphylline, theophylline, cromolyn sodium, nedocromil sodium, ketotifen, azelastine, ergotamine, cyclosporine, salmeterol, fluticasone, formoterol, procaterol, indacaterol, olodaterol, carmoterol, milveterol, vilanterol, abediterol, mepolizumab, omalizumab, montelukast, zafirlukast, betamethasone sodium phosphate, dexamethasone, dexamethasone sodium phosphate, dexamethasone acetate, prednisone, methylprednisolone acetate, zileuton, insulin, atropine, prednisolone, benzphetamine, chlorphentermine, amitriptyline, imipramine, clonidine, actinomycin c, bromocriptine, buprenorphine, pentamidine, calcitonin, leuprolide, alpha-1-antitrypsin, interferons, propranolol, lacicortone, triamcinolone, dinoprost, xylometazoline, diazepam, lorazepam, folic acid, nicotinamide, clenbuterol, ethinyloestradiol, levonorgestrel, and pharmaceutically acceptable salts and esters thereof such as albuterol sulfate, formoterol fumarate, salmeterol xinafoate, vilanterol trifenatate, beclomethasone dipropionate, triamcinolone acetonide, fluticasone propionate, fluticasone furoate, tiotropium bromide, leuprolide acetate and mometasone furoate.

Further drugs that may also be delivered by inhalation include but are not limited to aspirin, acetaminophen, ibuprofen, naproxen sodium, buprenorphine hydrochloride, propoxyphene hydrochloride, propoxyphene napsylate, meperidine hydrochloride, hydromorphone hydrochloride, morphine sulfate, fentanyl citrate, oxycodone hydrochloride, codeine phosphate, dihydrocodeine bitartrate, pentazocine hydrochloride, hydrocodone bitartrate, levorphanol tartrate, diflunisal, diamorphine, trolamine salicylate, methadone hydrochloride, nalbuphine hydrochloride, nalorphine, tetrahydrocannabinol, mefenamic acid, butorphanol tartrate, choline salicylate, butalbital, phenyltoloxamine citrate, diphenhydramine citrate, methotrimeprazine, cinnamedrine hydrochloride, meprobamate, ergotamine tartrate, propanolol hydrochloride, isometheptene mucate, dichloralphenazone, sumatriptan, rizatriptan, zolmitriptan, naratriptan, eletriptan, barbiturates (e.g., pentobarbital, pentobarbital sodium, secobarbital sodium), benzodiazapines (e.g., flurazepam hydrochloride, triazolam, tomazeparm, midazolam hydrochloride, lorazepam, buspirone hydrochloride, prazepam, chlordiazepoxide hydrochloride, oxazepam, clorazepate dipotassium, diazepam, temazepam), lidocaine, prilocaine, xylocaine, beta-adrenergic blockers, calcium channel blockers (e.g., nifedipine, diltiazem hydrochloride, and the like), nitrates (e.g., nitroglycerin, isosorbide dinitrate, pentaerythritol tetranitrate, erythrityl tetranitrate), hydroxyzine pamoate, hydroxyzine hydrochloride, alprazolam, droperidol, halazepam, chlormezanone, haloperidol, loxapine succinate, loxapine hydrochloride, thioridazine, thioridazine hydrochloride, thiothixene, fluphenazine hydrochloride, fluphenazine decanoate, fluphenazine enanthate, trifluoperazine hydrochloride, chlorpromazine hydrochloride, perphenazine, lithium citrate, prochlorperazine, lithium carbonate, bretylium tosylate, esmolol hydrochloride, verapamil hydrochloride, amiodarone, encainide hydrochloride, digoxin, digitoxin, mexiletine hydrochloride, disopyramide phosphate, procainamide hydrochloride, quinidine sulfate, quinidine gluconate, quinidine polygalacturonate, flecainide acetate, tocainide hydrochloride, lidocaine hydrochloride, phenylbutazone, sulindac, penicillamine, salsalate, piroxicam, azathioprine, indomethacin, meclofenamate sodium, gold sodium thiomalate, ketoprofen, auranofin, aurothioglucose, tolmetin sodium, colchicine, allopurinol, heparin, heparin sodium, warfarin sodium, urokinase, streptokinase, altoplase, aminocaproic acid, pentoxifylline, empirin, ascriptin, valproic acid, divalproate sodium, phenytoin, phenytoin sodium, clonazepam, primidone, phenobarbitol, phenobarbitol sodium, carbamazepine, amobarbital sodium, methsuximide, metharbital, mephobarbital, mephenytoin, phensuximide, paramethadione, ethotoin, phenacemide, secobarbitol sodium, clorazepate dipotassium, trimethadione, ethosuximide, doxepin hydrochloride, amoxapine, trazodone hydrochloride, amitriptyline hydrochloride, maprotiline hydrochloride, phenelzine sulfate, desipramine hydrochloride, nortriptyline hydrochloride, tranylcypromine sulfate, fluoxetine hydrochloride, doxepin hydrochloride, imipramine hydrochloride, imipramine pamoate, nortriptyline, amitriptyline hydrochloride, isocarboxazid, desipramine hydrochloride, trimipramine maleate, protriptyline hydrochloride, hydroxyzine hydrochloride, diphenhydramine hydrochloride, chlorpheniramine maleate, brompheniramine maleate, clemastine, azelastine, cyproheptadine hydrochloride, terfenadine citrate, clemastine, triprolidine hydrochloride, carbinoxamine maleate, diphenylpyraline hydrochloride, phenindamine tartrate, lamivudine, abacavir, acyclovir, gancyclovir, valganciclovir, cidofovir, foscarnet, azatadine maleate, tripelennamine hydrochloride, dexchlorpheniramine maleate, methdilazine hydrochloride, trimprazine tartrate, trimethaphan camsylate, phenoxybenzamine hydrochloride, pargyline hydrochloride, deserpidine, diazoxide, guanethidine monosulfate, minoxidil, rescinnamine, sodium nitroprusside, rauwolfia serpentina, alseroxylon, phentolamine mesylate, reserpine, calcitonin, parathyroid hormone, acitretin, amikacin sulfate, aztreonam, benzydamine, calcipotriol, chloramphenicol, chloramphenicol palmitate, chloramphenicol sodium succinate, ciprofloxacin hydrochloride, clindamycin hydrochloride, clindamycin palmitate, clindamycin phosphate, efalizumab, metronidazole, metronidazole hydrochloride, gentamicin sulfate, lincomycin hydrochloride, tobramycin sulfate, tacrolimus, vancomycin hydrochloride, polymyxin B sulfate, colistimethate sodium, colistin sulfate, tetracycline, griseofulvin, keloconazole, interferon gamma, zidovudine, amantadine hydrochloride, ribavirin, acyclovir, pentamidine e.g. pentamidine isoethionate, cephalosporins (e.g., cefazolin sodium, cephradine, cefaclor, cephapirin sodium, ceftizoxime sodium, cefoperazone sodium, cefotetan disodium, cefutoxime axotil, cefotaxime sodium, cefadroxil monohydrate, ceftazidime, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, cefuroxime sodium, and the like), penicillins (e.g., ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G potassium, penicillin G procaine, methicillin sodium, nafcillin sodium, and the like), erythromycins (e.g., erythromycin ethylsuccinate, erythromycin, erythromycin estolate, erythromycin lactobionate, erythromycin siearate, erythromycin ethylsuccinate, and the like), tetracyclines (e.g., tetracycline hydrochloride, doxycycline hyclate, minocycline hydrochloride, GM-CSF, ephedrine, pseudoephedrine, ammonium chloride, androgens (e.g., danazol, testosterone cypionate, fluoxymesterone, ethyltostosterone, testosterone enanihate, methyltestosterone, fluoxymesterone, testosterone cypionate), estrogens (e.g., estradiol, estropipate, conjugated estrogens), progestins (e.g., methoxyprogesterone acetate, norethindrone acetate), levothyroxine sodium, human insulin, purified beef insulin, purified pork insulin, glyburide, chlorpropamide, glipizide, tolbutamide, tolazamide, rosiglitazone, pioglitazone, troglitazone, clofibrate, dextrothyroxine sodium, probucol, lovastatin, rosuvastatin, niacin, DNase, alginase, superoxide dismutase, lipase, calcitonion, alpha-1-antitrypsin, interferons, sense or anti-sense nucleic acids encoding any protein suitable for delivery by inhalation, erythropoietin, famotidine, cimetidine, ranitidine hydrochloride, omeprazole, esomeprazole, lanzoprazole, meclizine hydrochloride, nabilone, prochlorperazine, dimenhydrinate, promethazine hydrochloride, thiethylperazine, scopolamine, sildenafil, vardenafil, cilomilast, imiquimod or resiquimod. Where appropriate, these drugs may be delivered in alternative salt forms.

Excipients may include for example, surfactants, co-solvent suspending aids, and/or particulate bulking agents.

Suitable surfactants include those disclosed in EP 372777, GB 837465 and GB 994734, each incorporated herein by reference. Span 85, oleic acid and/or lecithin are commonly used in medicinal aerosol formulations. Other suitable surfactants for use in medicinal aerosol formulations include HFA-soluble fluorocarbons such as those referred to in WO 91/11173, GB 2263064, each incorporated herein by reference, as well as polyethyleneoxide, polyoxyethylene-oxypropylene block copolymers such as members of the the Synperonic PE series (Croda International plc), polyoxypropylenes, polyoxyethylene-polyoxypropylene-ethylenediamine copolymers such as members of the Synperonic T series, castor oil ethoxylates such as Alakasurf CO-40, acetylated monoglycerides (e.g. Myvacet 9-40 or 9-45 from Farma International), polyvinyl pyrrolidone, polyvinylacetate, polyvinyl alcohol, polymers of acrylic acid, methacrylic acid and copolymers thereof, polyoxyethylene glyceryl trioleate (TagatTO), polyoxyethylene glyceryl monooleate (TagatO or TagatO2 from Degussa), diol-diacids such as those disclosed in WO 94/21228, incorporated herein by reference, oligolactic acid and derivatives thereof, such as those disclosed in WO 94/21229, incorporated herein by reference, functionalized PEGs such as those disclosed in WO 2003/059317, incorporated herein by reference, amide-ester excipients such as those disclosed in WO 2003/059331, incorporated herein by reference, propoxylated PEG (Antarox 31R1 from Solvay), polyoxyethylene glycerol esters such as those disclosed in US 5536444, incorporated herein by reference, protective colloids such as those described in WO 95/15151, incorporated herein by reference, glyceryl triesters, capr(yl)ic diglyceryl succinates (e.g. Miglyol 829 from Condea Chemie GmbH), Vitamin E acetate, tocopherol (Vitamin E), polyglycolized polyglyceride (e.g. Labrafac Hydro WL 1219 from Gattefosse, Gennevilliers, France), polypropylene glycol, polyethylene glycol e.g. PEG300, aminoacids or derivatives such as disclosed in US 6136294 incorporated herein by reference, and other surfactants in the same chemical family as the above but differing in chain length of alkyl or polyalkoxy groups.

Suitable co-solvents may include ethanol, propanol, isopropanol, and other alcohols, glycerol, polyethylene glycol 400, propylene glycol, decanol, sorbitol, mannitol, lactitol, maltitol, glycofurol, dipropylene glycol, propylene glycol diesters of medium chain fatty acids (e.g. Miglyol 840), triglyceride esters of medium chain fatty acids (e.g. Miglyol 810, 812), perfluorocyclobutane, perfluoropentane, perfluorodimethylcyclobutane, menthol, eucapyptus oil, propylene glycol monolaurate (Lauroglycol), diethylene glycol monoethyl ester (Transcutol), isopropyl myristate, saturated hydrocarbons in liquid form and essential oils. Ethanol is commonly used in medicinal aerosol formulations.

Suitable suspending aids may include lactose, glucose, sucrose, D(+)trehalose, as well as their various hydrates, anomers and/or enantiomers, other saccharides such as D-galactose, maltose, D(+)raffinose pentahydrate, sodium saccharin, polysaccharides such as starches, modified celluloses, dextrins, dextrans, DL-alanine, other aminoacids or derivatives such as disclosed in US 6136294 incorporated herein by reference, ascorbic acid, sodium sulphate, cetyl pyridinium chloride or bromide other salts e.g. sodium chloride, calcium carbonate, sodium tartrate, calcium lactate, or other organic compounds e.g. urea or propyliodone.

## Claims

1. A metered dose dispensing valve (202) for dispensing metered volumes of an aerosol formulation from an aerosol container (201), said valve comprising
a first valve body (213) defining in part a metering chamber (212);
a compliant biasing member (215);
a valve stem (214) passing axially through the metering chamber (212), movable relative to the chamber between non-dispensing and dispensing positions, and biased from its dispensing position towards its non-dispensing position by the compliant biasing member (215); and
a second valve body (230) defining at least in part a pre-metering region (223);
wherein said valve stem (214) and second valve body (230) are integrally provided in a single component (221), **characterized in that** the biasing member (215) is provided as a separate component and positioned relative to said valve-stem-second-valve-body-comprising component (221) such that at least one portion of the biasing member (215) engages the valve-stem-second-valve-body-comprising component (221) at a first position and
at least one other portion of the biasing member (215) engages the valve-stem-second-valve-body-comprising component (221) at a second position, thereby imparting a bias to the valve stem (214), and wherein, in use, the valve stem (214) is moved axially against said bias from its non-dispensing position into its dispensing position and upon release the valve stem (214) moves under the action of the bias from its dispensing position back to its non-dispensing position.

2. A metered dose dispensing valve (202) according to claim 1, wherein the at least one portion of the biasing member (215) engages the valve-stem-second-valve-body-comprising component (221) such that the at least one portion of the biasing member (215) is anchored relative to the valve-stem-second-valve-body-comprising component (221).

3. A metered dose dispensing valve (202) according to claim 1 or 2, wherein the second valve body (230) comprises a non-compliant portion (991) and a compliant portion (992).

4. A metered dose dispensing valve (202) according to claim 3, wherein at least one part of the compliant portion (992) of the second valve body (230) is joined either directly or indirectly to the valve stem (214) and at least one other part of the compliant portion (992) of the second valve body (230) is joined either directly or indirectly to the non-compliant portion (991) of the second valve body.

5. A metered dose dispensing valve (202) according to any one of the preceding claims, wherein the valve is configured and arranged such that, in use, when the valve stem (214) is in its non-dispensing position, the pre-metering region (223) is in communication either continuously or transiently with the contents of the aerosol container (201) and the metering chamber (212) is in communication at least transiently with the pre-metering region (223) to allow substance to pass from the aerosol container (201) to the metering chamber (212), and when the valve stem is in its dispensing position, the pre-metering region (223) is isolated from the metering chamber (212) and a communication path is provided between the metering chamber (212) and the outside of the valve and outside of an aerosol container assembly constituted by the valve and the aerosol container, said path being either continuously or transiently open to allow substance to pass from the metering chamber to the outside of the valve and aerosol container assembly,

6. A metered dose dispensing valve (202) according to any one of the preceding claims, wherein the valve is configured and arranged such that, in use, the valve stem (214) is moved axially inwardly towards the aerosol container (201) from its non-dispensing position to its dispensing position and upon release moves outwardly under the action of the bias from its dispensing position to its non-dispensing position.

7. A metered dose dispensing valve (202) according to any one of the preceding claims, wherein said compliant biasing member (215) is a spring member, in particular a compression or a tension spring member.

8. A metered dose dispensing valve (202) according to any one of claims 1 to 7, wherein the compliant biasing member (215) comprises a polymeric material, in particular a polymeric material selected from the group consisting of acetal (polyoxymethylene) polymers, polyetherimides, liquid crystalline polymers, polyetheretherketones, polyvinylidene difluorides, polycarbonates, polyethersulphones, phenolic laminates, as well as mixtures and composites thereof.

9. A metered dose dispensing valve (202) according to any one of the preceding claims, wherein said valve-stem-second-valve-body-comprising component (221) comprises a polymeric material, in particular a polymeric material selected from the group consisting of acetal (polyoxymethylene) polymers, polyetherimides, liquid crystalline polymers, polyetheretherketones, polyvinylidene difluorides, polycarbonates, polyethersulphones, phenolic laminates, as well as mixtures and composites thereof.

10. A metered dose dispensing valve (202) according to any one of the preceding claims, wherein the first valve body (213) has two open ends through which a valve stem passes, wherein relative to an affixed aerosol container (201), the first open end is located towards the interior of the container (201) and the second open end is located away from the interior of the container (201).

11. A metered dose dispensing valve (202) according to any one the preceding claims, wherein the valve further comprises an inner seal (216) and an outer seal (217), in particular relative to an affixed container (201), wherein the outer seal (217) is located at or near the open end of the first valve body (213) away from the interior of the container, and wherein the inner seal (216) is located at or near the open end of the first valve body (213) towards the interior of the container.

12. A metered dose dispensing valve (202) according to any one of the preceding claims, wherein the valve further comprises a ferrule (211).

13. A metered dose dispensing valve (202) according to any one of the preceding claims, wherein the valve further comprises a gasket seal (208).

14. A metered dose dispensing valve (202) according to any one of the preceding claims, wherein the compliant biasing member (215) is located partially within the pre-metering region (223) or completely within the pre-metering region.

15. A canister comprising an aerosol container (201) and a metered dose dispensing valve (202) according to any one of the preceding claims.

## Patentansprüche

1. Dosierventil (202) zur Abgabe von dosierten Mengen einer Aerosolrezeptur aus einem Aerosolbehälter (201), umfassend
einen ersten Ventilkörper (213), welcher zum Teil eine Dosierkammer (212) definiert;
ein nachgiebiges Vorspannungsbauteil (215);
einen Ventilschaft (214), welcher axial durch die Dosierkammer (212) hindurchgeht und relativ zur Kammer zwischen den Positionen "dosierend" und "nicht dosierend" beweglich ist, und aus seiner dosierenden Position in die nicht dosierende Position durch das nachgiebige Vorspannungsbauteil (215) beeinflusst wird; und
einen zweiten Ventilkörper (230), welcher mindestens zum Teil einen Vordosierbereich (223) definiert;
worin der genannte Ventilschaft (214) und der zweite Ventilkörper (230) integral in einem einzigen Bauteil (221) bereitgestellt werden, **dadurch gekennzeichnet, dass** das Vorspannungsbauteil (215) als ein separates Bauteil ausgebildet ist und relativ zum genannten aus Ventilschaft und zweitem Ventilkörper bestehenden Bauteil (221) dergestalt positioniert ist, dass mindestens ein Teil des Vorspannungsbauteils (215) in das aus Ventilschaft und zweitem Ventilkörper bestehende Bauteil (221) an einer ersten Position eingreift und mindestens ein anderer Teil des Vorspannungsbauteils (215) in das aus Ventilschaft und zweitem Ventilkörper bestehende Bauteil (221) an einer zweiten Position eingreift, wodurch eine Vorspannung auf den Ventilschaft (214) ausgeübt wird, und worin, bei Gebrauch, der Ventilschaft (214) axial gegen die genannte Vorspannung aus seiner nicht dosierenden Position in seine dosierende Position bewegt wird, und, bei Entspannung, der Ventilschaft (214) unter dem Einfluss der Vorspannung aus seiner dosierenden Position in seine nicht dosierende Position zurück bewegt wird.

2. Dosierventil (202) nach Anspruch 1, worin der zumindest eine Teil des Vorspannungsbauteils (215) derart mit dem aus Ventilschaft und zweitem Ventilkörper bestehenden Bauteil (221) in Eingriff ist, dass der mindestens eine Teil des Vorspannungsbauteils (215) relativ zu dem aus Ventilschaft und zweitem Ventilkörper bestehenden Bauteil (221) verankert ist.

3. Dosierventil (202) nach einem der Ansprüche 1 oder 2, worin der zweite Ventilkörper (230) einen nicht nachgiebigen Teil (991) und einen nachgiebigen Teil (992) umfasst.

4. Dosierventil (202) nach Anspruch 3, worin mindestens ein Teil des nachgiebigen Teils (992) des zweiten Ventilkörpers (230) direkt oder indirekt mit dem Ventilschaft (214) verbunden ist und mindestens ein anderer Teil des nachgiebigen Teils (992) des zweiten Ventilkörpers (230) entweder direkt oder indirekt mit dem nicht nachgiebigen Teil (991) des zweiten Ventilkörpers verbunden ist.

5. Dosierventil (202) nach einem der vorhergehenden Ansprüche, worin das Ventil derart konfiguriert und angeordnet ist, dass bei Gebrauch, wenn der Ventilschaft (214) sich in seiner nicht dosierenden Position befindet, der Vordosierbereich (223) entweder kontinuierlich oder vorübergehend mit dem Inhalt des Aerosolbehälters (201) kommuniziert und die Dosierkammer (212) zumindest vorübergehend mit dem Vordosierbereich (223) kommuniziert, damit Material aus dem Aerosolbehälter (201) in die Dosierkammer (212) gelangen kann, und wenn der Ventilschaft sich in der dosierenden Position befindet, der Vordosierbereich (223) von der Dosierkammer (212) isoliert ist und ein Kommunikationspfad zwischen Dosierkammer (212) und dem Außenbereich des Ventils und dem Außenbereich einer aus Ventil und Aerosolbehälter bestehenden Aerosolbehälteranordnung bereitgestellt wird, wobei der genannte Pfad entweder kontinuierlich oder vorübergehend geöffnet ist, um den Durchfluss von Material aus der Dosierkammer in den Außenbereich von Ventil und Aerosolbehälteranordnung zu ermöglichen,

6. Dosierventil (202) nach einem der vorangehenden Ansprüche, worin das Ventil derart konfiguriert und angeordnet ist, dass bei Gebrauch der Ventilschaft (214) axial nach innen hin zum Aerosolbehälter (201) aus seiner nicht dosierenden Position in seine dosierende Position bewegt wird und sich bei Entlastung unter dem Einfluss der Vorspannung nach außen, aus seiner dosierenden Position in die nicht dosierende Position, bewegt.

7. Dosierventil (202) nach einem der vorangehenden Ansprüche, worin das genannte nachgiebige Vorspannungsbauteil (215) ein Federbauteil, insbesondere eine Kompressionsfeder oder eine Spannfeder ist.

8. Dosierventil (202) nach einem der Ansprüche 1 bis 7, worin das nachgiebige Vorspannungsbauteil (215) ein Polymermaterial umfasst, insbesondere ein Polymermaterial ausgewählt aus der Gruppe bestehend aus Acetal-(Polyoxymethylen)-Polymeren, Polyetherimiden, flüssigkristallinen Polymeren, Polyetheretherketonen, Polyvinylidendifluoriden, Polycarbonaten, Polyethersulfonen, Phenol-Laminaten sowie Mischungen und Verbundstoffen daraus.

9. Dosierventil (202) nach einem der vorangehenden Ansprüche, worin das vorgenannte aus Ventilschaft und zweitem Ventilkörper bestehende Bauteil (221) ein Polymermaterial umfasst, insbesondere ein Polymermaterial ausgewählt aus der Gruppe bestehend aus Acetal-(Polyoxymethylen)-Polymeren, Polyetherimiden, flüssigkristallinen Polymeren, Polyetheretherketonen, Polyvinylidendifluoriden, Polycarbonaten, Polyethersulfonen, Phenol-Laminaten sowie Mischungen und Verbundstoffen daraus.

10. Dosierventil (202) nach einem der vorhergehenden Ansprüche, worin der erste Ventilkörper (213) über zwei offene Enden verfügt, durch die ein Ventilschaft hindurchgeht, wobei, relativ zu einem befestigten Aerosolbehälter (201), das erste offene Ende dem Inneren des Behälters (201) zugewandt angeordnet ist und das zweite offene Ende dem Inneren des Behälters (201) abgewandt angeordnet ist.

11. Dosierventil (202) nach einem der vorhergehenden Ansprüche, worin das Ventil ferner eine innere Dichtung (216) und eine äußere Dichtung (217) umfasst, insbesondere relativ zu einem befestigten Behälter (201), wobei die äußere Dichtung (217) am oder in der Nähe des offenen Endes des ersten Ventilkörpers (213) dem Inneren des Behälters abgewandt angeordnet ist, und wobei die innere Dichtung (216) am oder in der Nähe des offenen Endes des ersten Ventilkörpers (213) dem Inneren des Behälters zugewandt angeordnet ist.

12. Dosierventil (202) nach einem der vorhergehenden Ansprüche, worin das Ventil ferner eine Pressklemme (211) umfasst.

13. Dosierventil (202) nach einem der vorhergehenden Ansprüche, worin das Ventil ferner einen Dichtungsring (208) umfasst.

14. Dosierventil (202) nach einem der vorangehenden Ansprüche, worin das nachgiebige Vorspannungsbauteil (215) teilweise im Vordosierbereich (223) oder vollständig im Vordosierbereich angeordnet ist.

15. Kanister umfassend einen Aerosolbehälter (201) und ein Dosierventil (202) nach einem der vorhergehenden Ansprüche.

## Revendications

1. Soupape de distribution de dose mesurée (202) pour distribuer des volumes mesurés d'une formulation d'aérosol à partir d'un récipient pour aérosol (201), ladite soupape comprenant
un premier corps de soupape (213) définissant en partie une chambre de dosage (212) ;
un élément de sollicitation souple (215) ;
une tige de soupape (214) passant axialement à travers la chambre de dosage (212), mobile par rapport à la chambre entre des positions de non-distribution et de distribution, et sollicitée depuis sa position de distribution vers sa position de non-distribution par l'élément de sollicitation souple (215) ; et
un deuxième corps de soupape (230) définissant au moins en partie une région de pré-dosage (223) ;
dans laquelle ladite tige de soupape (214) et ledit deuxième corps de soupape (230) sont fournis de façon solidaire en un composant unique (221), **caractérisée en ce que** l'élément de sollicitation (215) est fourni en tant que composant indépendant et positionné par rapport audit composant comprenant tige de soupape-deuxième corps de soupape (221) de sorte qu'au moins une partie de l'élément de sollicitation (215) vient en prise avec le composant comprenant tige de soupape-deuxième corps de soupape (221) à une première position et au moins une autre partie de l'élément de sollicitation (215) vient en prise avec le composant comprenant tige de soupape-deuxième corps de soupape (221) à une deuxième position, en communiquant de ce fait une sollicitation à la tige de soupape (214), et dans laquelle, en cours d'utilisation, la tige de soupape (214) est déplacée axialement à l'encontre de ladite sollicitation depuis sa position de non-distribution dans sa position de distribution et lors de la libération de la tige de soupape (214) revient sous l'action de la sollicitation de sa position de distribution à sa position de non-distribution.

2. Soupape de distribution de dose mesurée (202) selon la revendication 1, dans laquelle ladite au moins une partie de l'élément de sollicitation (215) vient en prise avec le composant comprenant tige de soupape-deuxième corps de soupape (221) de telle sorte que ladite au moins une partie de l'élément de sollicitation (215) est ancrée par rapport au composant comprenant tige de soupape-deuxième corps de soupape (221).

3. Soupape de distribution de dose mesurée (202) selon la revendication 1 ou 2, dans laquelle le deuxième corps de soupape (230) comprend une partie non souple (991) et une partie souple (992).

4. Soupape de distribution de dose mesurée (202) selon la revendication 3, dans laquelle au moins une partie de la partie souple (992) du deuxième corps de soupape (230) est jointe ou directement ou indirectement à la tige de soupape (214) et au moins une autre partie de la partie souple (992) du deuxième corps de soupape (230) est jointe ou directement ou indirectement à la partie non souple (991) du deuxième corps de soupape.

5. Soupape de distribution de dose mesurée (202) selon l'une quelconque des revendications précédentes, où la soupape est conçue et disposée de sorte que, en cours d'utilisation, lorsque la tige de soupape (214) se trouve dans sa position de non-distribution, la région de pré-dosage (223) est en communication de manière ou continue ou transitoire avec le contenu du récipient pour aérosol (201) et la chambre de dosage (212) est en communication de manière au moins transitoire avec la région de pré-dosage (223) pour permettre à la substance de passer du récipient pour aérosol (201) à la chambre de dosage (212), et lorsque la tige de soupape se trouve dans sa position de distribution, la région de pré-dosage (223) est isolée de la chambre de dosage (212) et une voie de communication est fournie entre la chambre de dosage (212) et l'extérieur de la soupape et à l'extérieur d'un ensemble récipient pour aérosol constitué de la soupape et du récipient pour aérosol, ladite voie étant de manière ou continue ou transitoire ouverte pour permettre à la substance de passer de la chambre de dosage à l'extérieur de l'ensemble soupape et récipient pour aérosol.

6. Soupape de distribution de dose mesurée (202) selon l'une quelconque des revendications précédentes, où la soupape est conçue et disposée de sorte que, en cours d'utilisation, la tige de soupape (214) est déplacée axialement vers l'intérieur en direction du récipient pour aérosol (201) depuis sa position de non-distribution vers sa position de distribution et lors de la libération se déplace vers l'extérieur sous l'action de la sollicitation depuis sa position de distribution vers sa position de non-distribution.

7. Soupape de distribution de dose mesurée (202) selon l'une quelconque des revendications précédentes, dans laquelle ledit élément de sollicitation souple (215) est un élément ressort, en particulier un élément ressort de compression ou de tension.

8. Soupape de distribution de dose mesurée (202) selon l'une quelconque des revendications 1 à 7, dans laquelle l'élément de sollicitation souple (215) comprend un matériau polymère, en particulier un matériau polymère choisi dans le groupe constitué de polymères acétal (polyoxyméthylène), polyétherimides, polymères cristallins liquides, polyétheréthercétones, difluorures de polyvinylidène, polycarbonates, polyéthersulfones, stratifiés phénoliques, ainsi que des mélanges et composites de ceux-ci.

9. Soupape de distribution de dose mesurée (202) selon l'une quelconque des revendications précédentes, dans laquelle ledit composant comprenant tige de soupape-deuxième corps de soupape (221) comprend un matériau polymère, en particulier un matériau polymère choisi dans le groupe constitué de polymères acétal (polyoxyméthylène), polyétherimides, polymères cristallins liquides, polyétheréthercétones, difluorures de polyvinylidène, polycarbonates, polyéthersulfones, stratifiés phénoliques, ainsi que des mélanges et composites de ceux-ci.

10. Soupape de distribution de dose mesurée (202) selon l'une quelconque des revendications précédentes, dans laquelle le premier corps de soupape (213) a deux extrémités ouvertes à travers lesquelles passe une tige de soupape, dans laquelle, par rapport à un récipient pour aérosol attaché (201), la première extrémité ouverte est située en direction de l'intérieur du récipient (201) et la deuxième extrémité ouverte est située à l'écart de l'intérieur du récipient (201).

11. Soupape de distribution de dose mesurée (202) selon l'une quelconque des revendications précédentes, où la soupape comprend en outre un joint interne (216) et un joint externe (217), en particulier par rapport à un récipient attaché (201), dans laquelle le joint externe (217) est situé au niveau ou près de l'extrémité ouverte du premier corps de soupape (213) à l'écart de l'intérieur du récipient, et dans laquelle le joint interne (216) est situé au niveau ou près de l'extrémité ouverte du premier corps de soupape (213) en direction de l'intérieur du récipient.

12. Soupape de distribution de dose mesurée (202) selon l'une quelconque des revendications précédentes, où la soupape comprend en outre une virole (211).

13. Soupape de distribution de dose mesurée (202) selon l'une quelconque des revendications précédentes, où la soupape comprend en outre un joint d'étanchéité (208).

14. Soupape de distribution de dose mesurée (202) selon l'une quelconque des revendications précédentes, dans laquelle l'élément de sollicitation souple (215) est situé partiellement au sein de la région de pré-dosage (223) ou complètement au sein de la région de pré-dosage.

15. Réservoir comprenant un récipient pour aérosol (201) et une soupape de distribution de dose mesurée (202) selon l'une quelconque des revendications précédentes.
